# EUROPEAN PATENT APPLICATION

(11) **EP 3 346 264 A1**
(43) Date of publication of application: **11.07.2018**
(21) Application number: 16842322.6
(22) Date of filing: 01.09.2016
(51) Int. Cl.: G01N 27/327

(54) **ALLERGEN DETECTION APPARATUS USING ELECTROCHEMICAL DETECTION METHOD**

(30) Priority: 01.09.2015 KR 20150123800
(71) Applicant: Wonmedical Corp., Bucheon-si, Gyeonggi-do 14542 (KR)
(72) Inventor: LIM, Tae Kyu, Tokyo 164-0013 (JP)
(74) Representative: Morabito, Sara
(86) International application number: PCT/KR2016/009798
(87) International publication number: WO 2017/039356

(57) **Abstract**

The electrochemical sensor according to the present invention can precisely measure the current value of serotonin by preventing a decrease in sensitivity due to the background in the blood and thus can effectively diagnose allergy.

## Description

### BACKGROUND OF THE INVENTION

### Field of the Invention

The present invention relates to an allergen detection apparatus according to an electrochemical detection method which includes an electrochemical sensor including a working electrode in which serotonin oxidation occurs and confirms presence or concentration of serotonin by measuring oxidation current flowing when an oxidation potential of serotonin is applied, wherein the working electrode includes a coating layer having a negative charge, and inhibits approach of an interfering substance having a negative charge by electrostatic force of the coating layer.

Further, the present invention relates to an allergen detection method including treating a separated biological sample with the sensor; and measuring an oxidation current flowing when the oxidation potential of serotonin is applied.

### Background of the Related Art

Currently, allergies are the result of a malfunction of the immune system, which means that a substance that has little effect on an average person causes an abnormal hypersensitivity to certain people, such as urticaria, itching, runny nose, and cough. There are many kinds of allergenic substances such as food, air, pollen, mold, dust, and animals. Symptoms associated with allergic reactions include mild symptoms such as itching and cough, but severe cases can lead to death from asthma, seizures, and shock, and the reaction can occur in a very short time, and therefore, it is important to diagnose allergic reactions in advance.

When an allergic substance enters our body, an IgE antibody called Immuno Globulin E is produced. Since the IgE antibody is produced more in a person having an allergic condition than the normal person, the IgE antibody concentration in the blood can be found relatively higher.

The existing allergy test includes a prick test, and as a recent diagnostic method, a method of measuring the amount of IgE antibody in the serum (hereinafter referred to as "IgE amount" or "IgE antibody value") is used. The prick test is a test that uses a spoid to drop an antigenic substance into the arm and stab it with a needle and then examine the allergic reaction according to the presence or absence of the marker. The IgE level measuring method is a kind of a blood test, which is a test to check the allergic response to antigenic substances according to the level of IgE in the blood, including radioimmunoassay, nonradioactive immunoassay, multiple antigen stimulus test (MAST), and radioimmunoorbent test (RAST) using isotopes. However, in the case of the prick test, as there is a possibility of shock due to hypersensitivity, the prick test can be classified as a very dangerous method according to a person. The method of measuring the amount of IgE requires troublesome manipulation and a special technique and apparatus, and it costs a lot of time and cost to get a final diagnosis.

Unlike allergen diagnostics, which measure the amount of IgE in vivo, the detection of mediators in the blood enables rapid and accurate identification of allergen test results.

The mediator detection method is a method of identifying an allergen by detecting a chemical mediator detected when an allergic reaction occurs. Chemical mediators include a preformed mediator that undergoes a cross-linking reaction of IgE antibodies by allergens and is degranulated from basophils and mast cells, and a newly generated mediator produced by the reaction. Preformed mediators include histamine, serotonin, chymase, arylsulfatase, N-acetyl-β-D-glucosaminidase, etc. Newly generated mediators include prostaglandin, leukotriene, and thromboxane, or the like.

It is possible to provide a POCT allergen detecting apparatus that provides an allergic reaction to an allergen by electrochemically measuring serotonin in a chemical mediator detected when various allergens are added to whole blood of an allergic patient.

In order to overcome the disadvantages of the allergen diagnosis method for measuring the amount of IgE, the inventor of the present invention has developed an allergen detecting apparatus that selectively measures serotonin in the blood produced by the allergic reaction through electrochemical detection (Korean Patent Publication No. 2007-0117239).

### SUMMARY OF THE INVENTION

Therefore, the inventors of the present invention have found that the conventional electrochemical sensor has a problem that the result of diagnosis of allergens through electrochemical detection of serotonin in blood shows low correlation with the result of diagnosis based on the amount of IgE. In order to overcome the aforementioned problem, the inventors confirmed that using a sensor having working electrodes which include a coating layer having a negative charge may improve the accuracy of the diagnosis of allergy and completed the present invention.

One object of the present invention is to provide an electrochemical sensor which includes a working electrode in which serotonin oxidation occurs and confirms presence or concentration of serotonin by measuring oxidation current flowing when an oxidation potential of serotonin is applied, wherein the working electrode includes a coating layer having a negative charge, and inhibits approach of an interfering substance having a negative charge by electrostatic force of the coating layer.

Another object of the present invention is to provide a strip for detecting serotonin which includes a working electrode in which serotonin oxidation occurs when oxidation potential of serotonin is applied; and a reference electrode for applying a voltage, wherein the working electrode comprises a coating layer having a negative charge so as to inhibit access of an interfering substance having a negative charge by an electrostatic force.

Another object of the present invention is to provide a sensor for serotonin detection having one or more of strips.

Another object of the present invention is to provide a serotonin measuring kit having the sensor.

Another object of the present invention is to provide an allergen detection method which includes treating a separated biological sample with the sensor of any one of claims 1 to 13; and measuring an oxidation current flowing when the oxidation potential of serotonin is applied.

The sensor according to the present invention can exhibit high accuracy in the diagnosis of allergy since it can reduce the background due to the negatively charged interfering substance in the sample.

### BRIEF DESCRIPTION OF THE DRAWINGS

Further objects and advantages of the invention can be more fully understood from the following detailed description taken in conjunction with the accompanying drawings in which:
FIG. 1A is a schematic diagram showing a conventional electrochemical sensor for serotonin measurement in which a working electrode is not coated with Nafion.
FIG. 1B is a conceptual diagram of an electrochemical sensor showing a method of measuring a current value of serotonin.
   1: Voltage applying device;
   2: Recorder;
   3: Fine electrode;
   4: Working electrode;
   5: Control electrode;
   6: Reference electrode;
   7: Sample
FIG. 2 is a graph showing the current peak in a cyclic voltammogram of serotonin and an oxidation reaction formula of serotonin.
FIG. 3 is a graph showing the ascorbic acid-derived cyclic voltammogram at a sensor A including a working electrode coated with Nafion and a sensor B including a working electrode not coated with Nafion.
FIG. 4 shows a cyclic voltammogram in the presence of ascorbic acid and serotonin in a sensor including a Nafion coated working electrode.
FIG. 5 is a graph showing the circulating voltage-current curve of blood (cyclic voltammogram) (FIG. 5) in which a background reduction effect is confirmed in a sensor A including a working electrode coated with Nafion and a sensor B including a working electrode not coated with Nafion.
FIG. 6 shows the values of oxidation currents when serotonin oxidation potentials were applied to a blood containing an allergen-positive reaction and blood showing a negative reaction according to an IgE antibody value, in a sensor including a Nafion-coated working electrode.

### DETAILED DESCRIPTION OF THE PREFERRED EMBODIMENT

The preferred embodiments of the invention will be hereinafter described in more detail with reference to the accompanying drawings.

Embodiments of the present invention will be described in more detail hereinafter with reference to the accompanying drawings. The present invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, the shapes and sizes of respective elements may be exaggerated for clarity.

The first aspect of the present invention is to provide an electrochemical sensor which includes a working electrode in which serotonin oxidation occurs and confirms presence or concentration of serotonin by measuring oxidation current flowing when an oxidation potential of serotonin is applied, wherein the working electrode includes a coating layer having a negative charge, and inhibits approach of an interfering substance having a negative charge by electrostatic force of the coating layer.

The second aspect of the present invention is to provide a strip for detecting serotonin which includes a working electrode in which serotonin oxidation occurs when oxidation potential of serotonin is applied; and a reference electrode for applying a voltage, wherein the working electrode includes a coating layer having a negative charge so as to inhibit access of an interfering substance having a negative charge by an electrostatic force.

The third aspect of the present invention is to provide a sensor for serotonin detection having one or more of strips.

The fourth aspect of the present invention is to provide a serotonin measuring kit having the sensor.

The fifth aspect of the present invention is to provide an allergen detection method which includes treating a separated biological sample with the sensor; and measuring an oxidation current flowing when the oxidation potential of serotonin is applied.

Hereinbelow, the present invention will be described in a greater detail.

In an electrochemical sensor that includes a working electrode in which serotonin oxidation occurs and a reference electrode to which a voltage is applied and in which an oxidation current flowing when an oxidizing potential of serotonin is measured is electrochemically detected by serotonin in the blood. Here, the relevance with the result based on IgE quantity was not obtained.

The inventors of the present invention have found that background rise and sensitivity lowering problems occur due to the influence of electroactive species in biochemical reagents during electrochemical detection. That is, ascorbic acid, urea, and uric acid, which are present in a high concentration in the blood, are found to act as an obstructing agent in electrochemically detecting serotonin.

Therefore, the inventors of the present invention have developed an apparatus capable of diagnosing allergies with high accuracy without deterioration of sensitivity. Since the inhibitory substances such as ascorbic acid and uric acid are negatively charged, by applying the point that it is impossible to pass through the polymer membrane with the fixed charge and introducing the coating layer with the negative charge on the working electrode where the oxidation reaction of serotonin occurs, it is confirmed that background is reduced and allergies can be diagnosed at the same time, and the present invention has been completed.

"Allergy-inducing substances" or "allergens" are substances that have little effect on the average person, but cause abnormal irritation such as urticaria, itching, runny nose, and coughing only to certain people. Pollen, house dust, food, viruses, ticks, drugs and the like are kinds of allergen. Pollen also varies widely, including birch, oak, grass, and urticaria. In the present invention, as long as the allergic reaction occurs and the concentration of serotonin in the blood can be increased, there is no limitation on the kind of allergen inducing substance.

"Serotonin" is a kind of physiologically active amine which has a structure of 5-hydroxytryptamine (5HT) and is composed of a molecular formula of C10H12N2O. It is a kind of physiologically active amine, and a chemical mediator secreted from mast cells by the allergic reaction.

When an antigen (allergen) causing allergies initially invades the body, an IgE antibody is produced in the body. The IgE antibody is combined with a high-affinity receptor for IgE, FcεRI. Subsequently, when the same antigen repeatedly invades the body and the antigen binds to the IgE-high affinity receptor located on the surface of the mast cell, the mast cell initiates an activation signal and eventually releases secretion of the mediator serotonin to induce an allergic reaction.

The oxidation reaction of serotonin can occur at 0.2 V to 0.4 V, specifically at 0.3 V to 0.4 V.

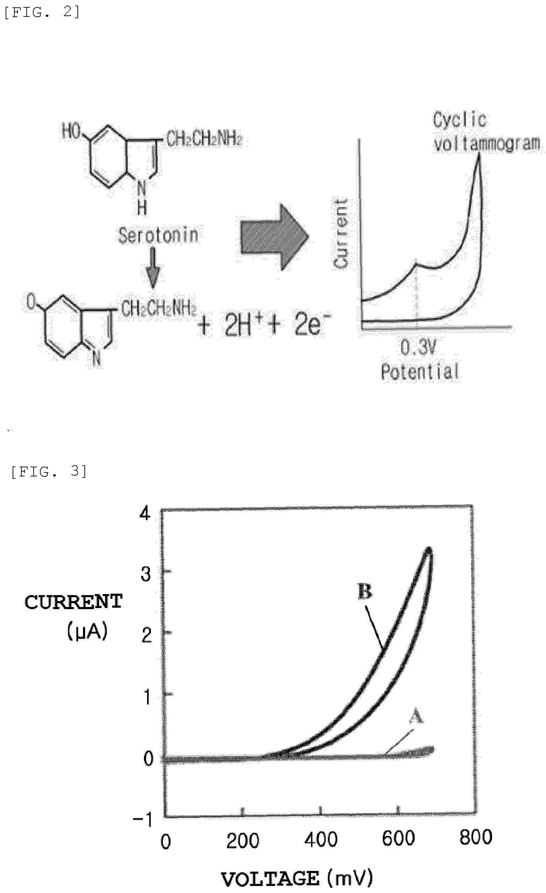

The electrode reaction of serotonin was measured by a cyclic voltammetry in the range of 0 to 1.0 V, and a current peak started to appear at 0.3 V (FIG. 2). The peak is represented by the electrons released from serotonin due to the oxidation of serotonin at 0.3 V. When the oxidation potential of 0.3 V is used, serotonin can be measured and the diagnostic possibility of the allergic reaction is confirmed.

In order to solve the problem of background rise and lowering of sensitivity in the oxidation reaction of serotonin in the working electrode due to the influence of ascorbic acid and / or urea and / or uric acid, which are electrode active species present in blood at high concentration, the working electrode includes a coating layer having a negative electric charge so as to suppress the approach of an interfering substance having a negative electric charge by an electrostatic force. The coating layer containing a polymer having a negative charge has a negative charge as well as an ion having a negative charge, such as ascorbic acid, urea or uric acid, due to the electrostatic force of the negative charge. The strips may allow access of ions having positive charges that are uncharged, neutral, or counter-charged while inhibiting access of the electrode active species.

Therefore, as long as the negative charged coating layer contains a polymer having a negative charge upon contact with the sample, there is no limitation on the kind of the material and the coating method.

For example, the coating layer may contain a positive charge ion exchange polymer. Non-limiting examples of the polymer include positively charged ion exchange resins, but may be used as a material for a coating layer as long as it is a material having a negative charge or a positively charged ion-exchange polymer and capable of coating a sensor. The polymer may include a sulfonic acid group (-SO3-), a carboxylic acid group (-COO-), an aldehyde group (-CHO), and a hydroxyl group (-OH).

As a non-limiting example, the positively charged ion-exchange polymer has a sulfonated tetrafluoroethylene based fluoropolymer-copolymer.

Nafion is a substance having ion-exchange properties and is a kind of "sulfonated tetrafluoroethylene based fluoropolymer-copolymer."

In one embodiment of the present invention, using a sensor including a working electrode coated with a negatively charged substance and a sensor including a non-coated working electrode, cyclic voltammetry of ascorbic acid was performed. As a result, in the sensor including the working electrode coated with the negatively charged substance, the current value deriving from the ascorbic acid did not appear, whereas in the sensor including the uncoated working electrode, a current value deriving from ascorbic acid in a range of 400 to 600 mV appears (FIG. 3). Through this, when the working electrode contains a coating layer having a negative charge, it is confirmed that the sensitivity is improved when the current value of serotonin is measured by inhibiting the access of the negatively charged interfering substance such as ascorbic acid.

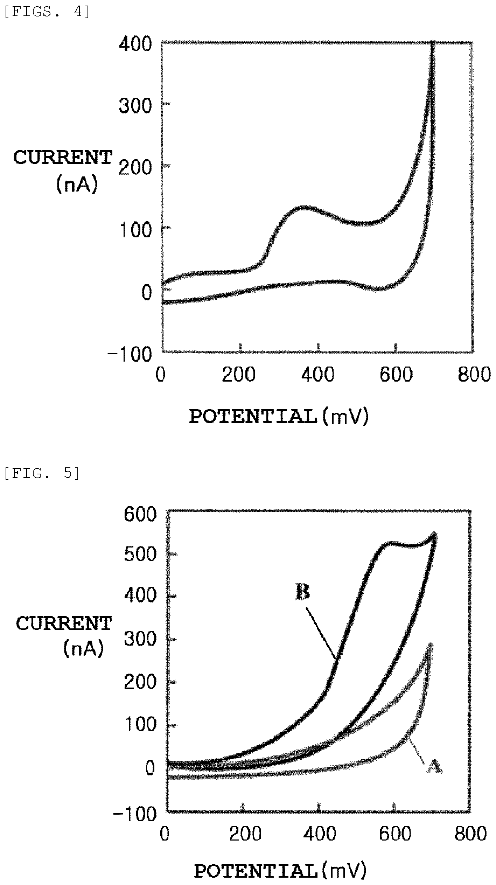

In addition, when ascorbic acid and serotonin were present together in the sample, the peak current of serotonin was measured using a sensor including a working electrode coated with a negatively charged substance. As a result, unlike the sensor, which contained a working electrode that was not coated with a negatively charged substance, at 350 mV, which measured serotonin, the current value appeared and the background peak of ascorbic acid did not appear (FIG. 4). As a result, it was confirmed that the sensor can measure serotonin without lowering the sensitivity.

For example, the coating layer may contain 0.001 to 99.9% by weight, specifically 0.01 to 95% by weight, of a polymer having a negative charge or a positively charged ion-exchange polymer.

For example, the coating layer may be formed with pores or patterns so that serotonin can directly contact the working electrode. For example, the electrode pattern of the working electrode and the coating layer pattern may be alternately formed without overlapping some or all of them, or the coating layer pattern may be formed thereon corresponding to the electrode pattern.

The working electrode of the present invention is an electrode in which the oxidation reaction of serotonin occurs.

Non-limiting examples of the working electrode material include metals such as copper, platinum, silver, gold, palladium, ruthenium, rhodium and iridium, carbon, semiconductors such as GaAs, CdS and In2O3, and the like. The best thing is the gold electrode. The working electrode and the reference electrode may be in the form of a printed circuit board.

In the meantime, the potential of the standard hydrogen electrode is referred to as 0 V for the sake of convenience, and a single electrode potential such as a calomel electrode or silver-silver chloride electrode can be generally used as a reference electrode. The reference electrode is preferably an Ag / AgCl electrode capable of maintaining a constant potential or an electrode usable as a similar reference electrode.

The electrochemical sensor according to the present invention may further include a control electrode for measuring a current change in addition to a working electrode and a reference electrode.

According to the present invention, there is provided a working electrode in which an oxidation reaction of serotonin occurs when an oxidation potential of serotonin is applied; a reference electrode to which a voltage is applied; and a control electrode for optionally measuring the current change may be formed on the strip. The strip may be separated or exchanged after one or more uses.

The strip of the present invention may be constituted by each electrode, and an electrical connection line connecting each electrode with the measurement device. An insulating layer may be formed using an insulating material in the remaining portion except for the electrode.

The sensor according to the present invention may be provided with two or more strips each having a working electrode and at least two electrodes of a reference electrode, so that two or more allergic reactions can be diagnosed.

The strip according to the present invention preferably has electrodes formed on a support made of a nonconductive insulating material. Such a support is preferably made to have a thickness of 20 to 60 microns, more preferably a thickness of 30 microns.

As the material of the support made of the nonconductive insulating material, any insulator can be used, but it is suitable to have some degree of flexibility and rigidity as a support for mass production. In general, it is preferable that the surface of the support is formed very evenly. This is because an uneven surface causes non-uniformity of the electrode surface area between each sensor strip in mass production, resulting in non-uniformity of the sensor output signal.

The material having the most even surface can be a silicon wafer used for manufacturing semiconductors. Next, a quartz glass substrate or a general glass substrate which is transparent and easy to process can be used. In the meantime, a general music compact disc has a very even surface and excellent flatness due to the characteristics of use, and it has a circular shape similar to a semiconductor wafer, so that a semiconductor manufacturing process equipment can be used. At the same time, it is cheaper and easier to obtain. In addition, ordinary plastic films can be used.

Examples of the compact disc or plastic film material include a plastic film such as a poly ester, a polycarbonate, a poly styrene, a polyimide, a poly vinyl chloride, a polyethylene poly ethylene, polyethylene terephthalate and the like.

Preferably, the electrodes on the strip according to the present invention each independently have a length of 14 mm to 19 mm, a width of 0.5 mm to 2 mm and a thickness of 20 to 150 microns, more preferably a length of 14 mm, a width of 1 mm, and thickness of 60 microns.

The electrodes of the present invention are preferably divided into a portion that directly contacts the sample and a portion that transmits a signal to the detector through an insulating coating, but the present invention is not limited thereto.

In the present invention, a "sample" applied to the diagnosis of allergy is a sample to be analyzed for the presence or secretion of serotonin, and includes a whole blood, a blood cell, a serum, a plasma, marrow, sweat, urine, tears, saliva, skin, mucous membranes, etc. of a mammal, and preferably a human, and may be, for example, blood. The sample may include a component (s) (e.g., mast cell) capable of secreting serotonin upon contact with a specific allergen in the case of a sample derived from a subject sensitized to a specific allergen. Alternatively, by treating allergens to induce serotonin secretion and then removing some or all of the cells involved in secreting serotonin is included. In addition, the sample may be an allergen-treated biological sample, and may be a biological sample before the allergen is treated.

The sensor according to the present invention may include a reaction vessel capable of receiving a sample.

The reaction vessel is configured such that a test sample or the like is selectively brought into contact with the working electrode and the reference electrode thereof, and when the liquid sample is introduced, the electrodes are energized to form a circuit. If the measurement sample is not a liquid, it may be dissolved in a solvent such as water and then introduced into a reaction vessel.

A shape of the reaction vessel is enough to be a structure capable of holding the sample and the measuring reagent and passing current between the electrodes after the introduction of the sample, and includes a working electrode, a reference electrode having a size capable of accommodating the reference electrode. The material of the reaction vessel can be used without restriction as long as it is electrically inactive and / or inactive to the sample or the electrode, such as a fiber aggregate like a filter paper, a nonwoven fabric, a porous material and a gel. Among them, polyvinyl chloride, polyimide, gelatin, glass fiber and the like can be included.

The reaction vessel may contain a pH buffering reagent as a measurement reagent. In addition, interference cancellation reagents may be included in the reaction vessel to remove interfering components that interfere with electrochemical measurements.

When the sample is introduced into the reaction vessel, the working electrode and the reference electrode (selectively, the control electrode) can be energized through the sample.

The reaction vessel can be held so as to absorb and retain the sample therein, and to be disposed after a single use. It is preferable that the nonwoven fabric is contained in the reaction vessel.

The reaction vessel may be filled with or coated with an allergen inducing substance. This is for reacting with the sample introduced into the reaction vessel and the allergen causing substance. The type of the receiving or coating and the concentration of the allergen causing substance may be changed depending on the sample.

The electrochemical sensor according to the present invention may have one reaction vessel or two or more reaction vessels for measuring allergic reactions to different allergens. Furthermore, each reaction vessel may contain a different allergen contained or coated.

In addition, the electrode pairs of the working electrode, the reference electrode and the control electrode are separately housed in separate reaction vessels, and each reaction vessel can treat each or all of the same or different allergy sources, and it may be that allergen sources are accommodated / coated in each reaction vessel.

For example, the electrochemical sensor according to the present invention includes a first working electrode and a first reference electrode for measuring serotonin for a first allergy source, and a second working electrode and a second reference electrode for measuring serotonin for a second allergy source, and an n-th working electrode and n-th reference electrode (n = an integer of one or more) for measuring serotonin for the n-th allergy source, and each electrode pair may be connected to an ammeter, and measure the current generated between the electrode pairs. Thus, an allergic reaction test can be performed on various allergen sources simultaneously.

Meanwhile, the sensor may include an automatic potential adjusting device for applying a potential to the reference electrode. The automatic potential adjusting device can automatically adjust the potential in the range of 0 to 1.0 V. Since the sensor of the present invention requires the application of the potential necessary for the oxidation reaction of serotonin, it is necessary to automatically adjust the potential between 0 and 1.0 V to include a voltage at which the oxidation reaction of serotonin can take place, for example, 0.3V.

In addition, the sensor of the present invention may further include a display unit for displaying a current value measured at the time of application of a potential at which the oxidation reaction of serotonin occurs.

The allergen identification method of the present invention may include a first step of treating a separated biological sample to the sensor, the strip, or the kit; and a second step of measuring the oxidation current flowing when the oxidation potential of serotonin is applied.

For example, the method may further include a third step of determining the sample as an allergen when a peak current appears in the range of 300 to 400 mV, specifically 350 mV, as measured in the second step.

### [Modes for the Invention]

Hereinafter, the constitution and effects of the present invention will be described in more detail with reference to Examples and Experimental Examples. These Examples and Experimental Examples are only for illustrating the present invention, and the scope of the present invention is not limited by these Examples and Experimental Examples.

### Reference Example 1: Measurement of current by oxidation reaction of serotonin

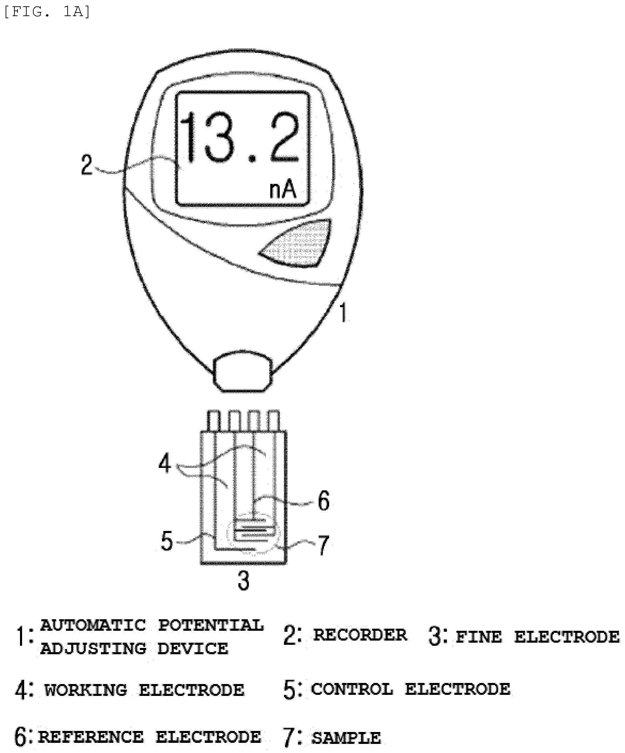

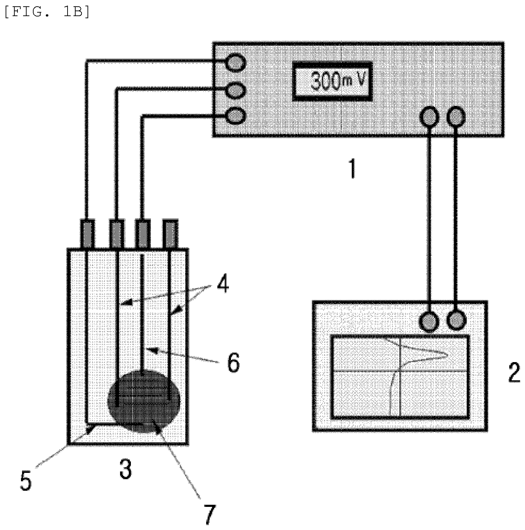

First, in order to detect serotonin, an electrochemical detection method was performed on the blood added with 1 µg / ml of serotonin using the apparatus shown in FIG. 1A. As a result of measuring electrode reaction with a constant speed (10mV / min) for unit time of 0 to 1.0 V versus Ag / AgCl, a general form of a cyclic voltammogram as shown in FIG. 2 was derived. In particular, the peak value obtained from 0.3V vs. Ag/AgCl means that the released electrons are transferred and the oxidation reaction occurs electrochemically. Therefore, the serotonin concentration can be quantified by measuring the current value at this time. That is, in order to quantitatively measure the concentration of serotonin, it is possible to derive the correlation with the current value with respect to the concentration. Such a measurement can be performed using the apparatus of FIG. 1B.

Specifically, when the sample 7 is loaded at a voltage applying device 1 for applying a potential to a reference electrode; and the sensor having the reference electrode 6 to which the voltage is applied, if serotonin is generated through the reaction between the sample and the allergen, the oxidation reaction of serotonin occurs in the working electrode 4, the current change is measured in the control electrode 5, and the measured result is displayed in the recorder 2.

### Exemplary embodiment 1: Electrochemical sensor with working electrode coated with Nafion

The working electrode (B) of the electrochemical sensor for serotonin measurement described in Korean Patent Publication No. 2007-0117239 shown in FIG. 1A was coated with Nafion. Specifically, 5 ml of a 5% by weight Nafion solution was dropped onto the electrode surface and dried at room temperature for 1 hour to prepare an electrochemical sensor having a working electrode (A) coated with Nafion.

### Exemplary embodiment 2: Synthesis of ascorbic acid Cyclic voltammetry of ascorbic acid

Using a sensor including each of the working electrode (A) coated with Nafion and the working electrode (B) not coated with Nafion according to exemplary embodiment 1, for a solution of 0.1 M phosphate buffer containing 100 mg / ml ascorbic acid, cyclic voltammetry of ascorbic acid was performed and a current potential curve (FIG. 3) was obtained. As a result, when the electrode (B) without the Nafion coating was used, the background was increased by the ascorbic acid, whereas when the Nafion coating electrode (A) was used, increase in background by ascorbic acid and increase in current value derived from ascorbic acid have not been confirmed.

### Exemplary embodiment 3: Ascorbic acid and cyclic voltammetry of serotonin

After adding ascorbic acid and serotonin to the sample (whole blood) at a concentration of 100 mg / ml and 1 µg / ml, respectively, the working electrode A coated with Nafion according to Exemplary embodiment 1 was used and cyclic voltammetry was performed on the whole blood. As a result, a peak current derived from serotonin was confirmed at around 350 mV (FIG. 4).

### Exemplary embodiment 4: Cyclic voltammetry of whole blood

Cyclic voltammetry was performed on whole blood without serotonin using a non-Nafion-coated electrode (B), and a high background was observed (FIG. 5). In contrast, it was confirmed that the background was reduced by using the electrode (A) coated with Nafion according to Exemplary embodiment 1 (FIG. 5) .

From the above results, it was suggested that by using Nafion coated electrodes, it is possible to inhibit the electrode interfering substances in whole blood, enabling selective detection of serotonin, and stable detection and quantification with high sensitivity of allergic reaction is possible.

### Exemplary embodiment 5: Correlation between IgE antibody value and electrochemical detection value

In order to measure the serotonin according to the allergic reaction, the specificity for allergen using the Nafion coated electrode according to Exemplary embodiment 1 was examined.

First, using an electrochemical sensor having an electrode (A) according to Exemplary embodiment 1 was tested for the whole blood of a patient diagnosed with a pine allergen and a tick allergen by measuring IgE antibody value (blood IgE concentration), electrochemical detection of allergic reactions was performed. As a result, 40 minutes after allergen addition, the current value was 38.6 nA for pine allergen and 130.3 nA for tick allergen was identified.

In addition, electrochemical detection was carried out using whole blood of a person who is negative for pine allergen but positive for tick allergen based on the value of IgE antibody. As a result, it showed 0.6 nA for pine allergen but 86.8 nA for tick allergen.

On the other hand, 0.7 nA and 0.4 nA, respectively, were found for whole blood of persons diagnosed as negative for pine allergen and tick allergen.

As described above, the electrochemical detection of the allergic reaction using the sensor equipped with the Nafion coated electrode shows that selective detection of allergen is possible because the correlation between the IgE antibody value and the pine allergen and mite allergen is significant. (Table 1)

**[Table 1]**

| Type | Gender (Age) | Types of Allergen | Current Value (nA) | IgE Antibody Value (UA/ml) |
|---|---|---|---|---|
| a | Female (30) | Pine | 38.6 | 4.70 |
| | | Mite | 130.3 | 60.00 |
| b | Male (24) | Pine | 0.6 | 0.34< |
| | | Mite | 86.8 | 48.06 |
| c | Male (24) | Pine | 0.7 | 0.34< |
| | | Mite | 0.4 | 0.34< |

From the above results, it was confirmed that when the IgE antibody value is high, the serotonin current value is high, and when the IgE antibody value is low, the serotonin current value is low. Further, it was confirmed that the diagnosis results based on the current value of serotonin in blood measured using the sensor including the working electrode coated with Nafion and the diagnosis result by the IgE antibody value showed the same pattern. This indicates that an allergic reaction can be confirmed by using a sensor including a working electrode coated with a polymer which is negatively charged without measuring the IgE antibody value.

### Exemplary embodiment 6: Confirmation of reproducibility of electrochemical detection value

In measuring the peak current of serotonin using a sensor including a Nafion coated working electrode, it was examined whether the measured peak current value is constant. Specifically, by measuring the IgE antibody value, the electrochemical measurement was carried out using a sensor including a working electrode (A) coated with Nafion of Exemplary embodiment 1 for an experimenter who was positive for pine allergen and a whole blood of a negative experimenter five times, and the reproducibility of the measured values was confirmed.

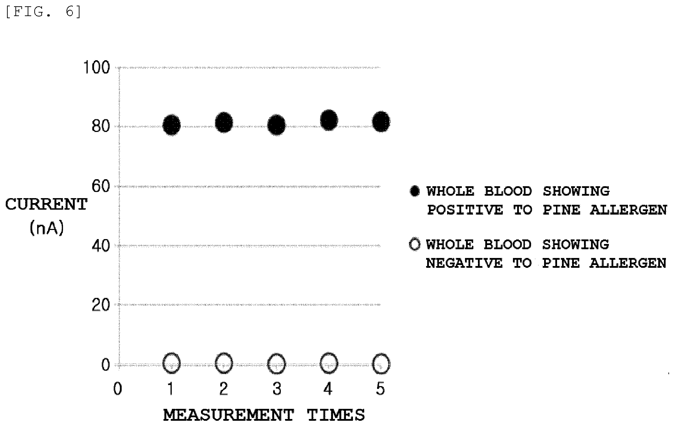

As a result of measurement, blood having an allergen-positive reaction according to the IgE antibody value showed a current peak of 80 nA or more, and the error of the result of five times of measurement was within 1 nA. In addition, even in the case of the blood exhibiting an allergic negative reaction according to the IgE antibody value, the error of the result of the 5-times measurement was within the range of 1 nA (FIG. 6). It was confirmed that the reproducibility of the measured values obtained from the electrochemical detection of the allergic reaction was high.

### Exemplary embodiment 7: Confirmation of correlation between IgE antibody value and electrochemical detection value through clinical experiment

Clinical experiments were conducted on 15 persons aged from 20 to 30 years old, and tick-related allergens were measured using an electrochemical detection method (using a sensor including a working electrode (A) coated with Nafion in Exemplary embodiment 1). The current value was compared with the IgE antibody value measured by an inspection institution, and the results are shown in Table 2.

**[Table 2]**

| Group | Gender (Age) | Current Value(nA) | IgE Antibody Value (UA/ml) | +/- |
|---|---|---|---|---|
| A | M (23) | 707.1 | 86.1 | + |
| | F (33) | 492.6 | 60.0 | + |
| | F (24) | 399.1 | 48.6 | + |
| | F (25) | 82.1 | 10.0 | + |
| | M (25) | 81.3 | 9.9 | + |
| | M (28) | 56.6 | 6.9 | + |
| | M (35) | 10.7 | 1.3 | + |
| B | M (36) | -0.2 | 0.34< | - |
| | M (29) | 1.1 | 0.34< | - |
| | F (26) | -0.7 | 0.34< | - |
| | F (29) | 0.3 | 0.34< | - |
| | M (23) | -0.7 | 0.34< | - |
| | F (23) | -4.8 | 0.34< | - |
| C | M (24) | 13.1 | 0.34< | ±, + |
| | F (26) | 12.3 | 0.34< | ±, + |

In the case of group A, both the diagnosis results based on the electrochemical detection method and the IgE antibody value were positive.

In the case of group B, the diagnosis result by the electrochemical detection method, the diagnosis result by the IgE antibody value, and the symptom were all negative.

In case of the group C, IgE antibody value was equal to or less than 0.34 (negative), while the current values by electrochemical detection showed a positive response. Furthermore, positive symptoms were found in the patients, and it was expressed as similar symptoms (± pseudopositive symptoms).

Therefore, it has been found that the detection method according to the present invention is very effective in diagnosing the allergen by the electrochemical detection method through the results of the groups A to C.

While the present invention have been described in connection with the exemplary embodiments illustrated in the drawings, it will be appreciated that they are merely an illustrative embodiments and various equivalent modifications and variations of the embodiments can be made by a person having an ordinary skill in the art without departing from the spirit and scope of the present invention. Therefore, the appended claims also include such modifications and variations falling within the true technical scope of the present invention.

## Claims

1. An electrochemical sensor which includes a working electrode (4, A, B) in which serotonin oxidation occurs and confirms presence or concentration of serotonin by measuring oxidation current flowing when an oxidation potential of serotonin is applied, wherein the working electrode (4, A, B) comprises a coating layer having a negative charge, and inhibits approach of an interfering substance having a negative charge by electrostatic force of the coating layer.

2. The sensor of claim 1, wherein the oxidation reaction of serotonin occurs between 0.3 to 0.4 V.

3. The sensor of claim 1, wherein the coating layer contains a positive charge ion-exchange polymer.

4. The sensor of claim 3, wherein the positive charge ion-exchange polymer is sulfonated tetrafluoroethylene based fluoropolymer-copolymer.

5. The sensor of claim 1, wherein the coating layer is formed with pores or patterns so that serotonin is capable of contacting a working electrode (4, A, B).

6. The sensor of claim 1, wherein the interfering substance is at least one selected from a group consisting of ascorbic acid, urea, and uric acid.

7. The sensor of claim 1, wherein the working electrode (4, A, B) and a reference electrode (6) are made of gold (Au) or carbon (C).

8. The sensor of claim 1, wherein a control electrode (5) for measuring a current change is included.

9. The sensor of claim 1, wherein a reaction vessel capable of accommodating a sample (7) is provided, and when a sample (7) is introduced to the reaction vessel, the working electrode (4, A, B) and the reference electrode (6) are energized to form a circuit.

10. The sensor of claim 9, wherein the reaction vessel is a vessel in which an allergy-inducing substance is contained or coated.

11. The sensor of claim 1, wherein the sensor comprises an automatic potential control device for applying a voltage of a desired potential to the reference electrode (6).

12. The sensor of claim 11, wherein the automatic potential control device automatically controls within a range of 0 to 1.0 V.

13. The sensor of claim 1, further comprising:
a display (2) for displaying current value measured at the time of application of the potential at which the oxidation reaction of serotonin occurs.

14. A strip for detecting serotonin comprising:
a working electrode (4, A, B) in which serotonin oxidation occurs when oxidation potential of serotonin is applied; and a reference electrode (6) for applying a voltage, wherein the working electrode (4, A, B) comprises a coating layer having a negative charge so as to inhibit access of an interfering substance having a negative charge by an electrostatic force.

15. The strip of claim 14, wherein the strip is able to be separated and exchanged so that one or a plurality of strips are able to be used.

16. A sensor for detecting serotonin having one or more of strips of claim 14 or 15.

17. A serotonin measuring kit having a sensor of any one of claims 1 to 13.

18. An allergen detection method comprising:
treating a separated biological sample with the sensor of any one of claims 1 to 13; and measuring an oxidation current flowing when the oxidation potential of serotonin is applied.
